# EUROPEAN PATENT SPECIFICATION

(11) **EP 3 020 331 B1**
(45) Date of publication and mention of the grant of the patent: **08.01.2020**
(21) Application number: 15193143.3
(22) Date of filing: 05.11.2015
(51) Int. Cl.: A61B 5/024, A61B 5/11, A61B 5/00, G06F 3/00

(54) **IMPROVING WRIST DEVICE EFFICIENCY**
VERBESSERUNG DER EFFIZIENZ EINER HANDGELENKSVORRICHTUNG
AMÉLIORATION DE L'EFFICACITÉ D'UN DISPOSITIF DE POIGNET

(30) Priority: 14.11.2014 US 201414542379
(43) Date of publication of application: 18.05.2016
(73) Proprietor: Polar Electro Oy, 90440 Kempele (FI)
(72) Inventor: Korkala, Seppo, 90440 Kempele (FI); Majava, Ville, 90440 Kempele (FI); Orava, Matti, 90440 Kempele (FI); Putila, Veli-Pekka, 90440 Kempele (FI); Isopoussu, Ari, 90440 Kempele (FI); Haapakoski, Heli, 90440 Kempele (FI)
(74) Representative: Kolster Oy Ab

(56) References cited:
- WO-A2-2012/061438
- KR-A- 20140 077 299
- US-A1- 2011 066 051
- US-A1- 2013 050 587
- US-A1- 2014 035 761
- US-A1- 2014 176 866

## Description

### FIELD OF THE INVENTION

The invention relates generally to physical activity measurement. More particularly, the present invention relates to using a wrist device to measure user's physical activity.

### BACKGROUND OF THE INVENTION

Measuring physical activity has become more and more popular, not only during training, but also when users are performing their everyday activities. Thus, a demand for solutions making the physical activity monitoring more effortless has risen. WO/2012/061438 discloses a wearable device assembly that has a housing supporting a controller, display and indicator system thereon. The controller has at least one sensor wherein activity of a user wearing the device is detected. The controller selectively illuminates the indicator system to indicate a level of activity of the user. KR2014/0077299 discloses LED matrix and colour conversion member comprising a fluorescent layer. Image is projected on a windshield. US2014/176866 discloses a LED device that includes an LED chip, a fluorescent layer and a lens. The fluorescent layer has phosphor powder diffused therein. The lens covers the LED chip and is positioned between the LED chip and the fluorescent layer. Light emitting from the LED chip travels through the lens and the fluorescent layer to obtain white light. The present discloser also provides a liquid crystal display using the LED device described above.

### BRIEF DESCRIPTION OF THE INVENTION

According to an aspect, there is provided a wrist device comprising: a curved body arranged to fit the wrist device around a user's wrist; an activity tracker circuitry configured to measure physical activity of the user of the wrist device; a processing circuitry configured to acquire physical activity-related measurement data from the activity tracker circuitry and to process the physical activity-related measurement data into physical activity-related metric characterizing the physical activity of the user; and a user interface element configured to display the physical activity-related metric, wherein the user interface element comprises: a light emitting diode matrix layer comprising a plurality of light emitting diodes arranged in a matrix form; and a fluorescent layer configured to alter the wavelength of the light emitted by the light emitting diode matrix layer, wherein the light emitting diode matrix layer comprises: a first layer electrically coupled to an electrical terminal, a second layer comprising the plurality of light emitting diodes, wherein the plurality of light emitting diodes are electrically coupled with the first layer, and a third layer electrically coupled with the plurality of light emitting diodes, wherein the second layer comprises electrical lead-throughs electrically coupling the first layer and the third layer, and wherein at least one of the first layer, the second layer, and the third layer is manufactured by utilizing electronics printing technique.

In an embodiment, the user interface element further comprises a cover layer arranged to be the top layer of the user interface element.

In an embodiment, the cover layer comprises a decoration layer arranged to be the bottom layer of the cover layer, and wherein the decoration layer comprises a visual decoration.

Although not claimed, in an example, the visual decoration is a dimming arranged to dim the light emitted by the light emitting diodes matrix layer.

Although not claimed, in an example, the cover layer comprises a lens layer arranged to be situated between the decoration layer and the hardened layer.

In an embodiment, the user interface element comprises a touch-screen layer comprising a touch-screen element, and wherein the touch-screen layer is arranged to be situated between the light emitting diode matrix layer and the cover layer.

Although not claimed, in an example, the touch-screen is a capacitive touch screen

Some further embodiments are defined in the dependent claims.

### BRIEF DESCRIPTION OF THE DRAWINGS

Embodiments of the present invention are described below, by way of example only, with reference to the accompanying drawings, in which
Figure 1 illustrates a physical activity measurement system to which embodiments of the invention may be applied;
Figure 2A illustrates a wrist device of the physical activity measurement system according to an embodiment of the invention;
Figure 2B illustrates measuring of activity according to an embodiment of the invention;
Figures 2C and 2D illustrate embodiments of the invention;
Figure 3 illustrates the user interface element according to an embodiment of the invention;
Figure 4 illustrates an embodiment of the invention;
Figure 5A illustrates the user interface element according to an embodiment of the invention;
Figure 5B illustrates a optical conductor layer of the user interface element according to an embodiment of the invention; and
Figure 6 shows a block diagram of the wrist device according to an embodiment of the invention.

### DETAILED DESCRIPTION OF THE INVENTION

The following embodiments are exemplary. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments. Furthermore, words "comprising" and "including" should be understood as not limiting the described embodiments to consist of only those features that have been mentioned and such embodiments may contain also features/structures that have not been specifically mentioned.

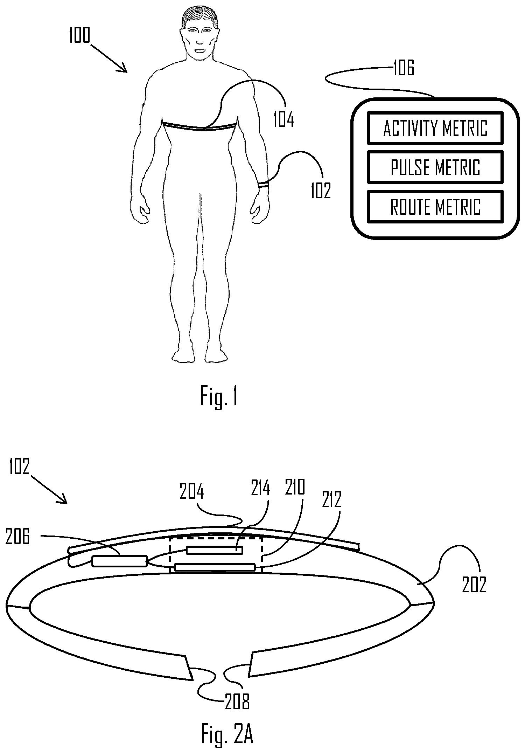

Figure 1 illustrates a physical activity measurement system to which embodiments of the invention may be applied. Referring to Figure 1, physical activity of a user 100 may be monitored using an activity tracker apparatus 102. The activity tracker apparatus 102 may be a portable or wearable electronic device, such as a wrist device 102. The wrist device 102 may comprise an optical heart activity sensor configured to measure user's 100 heart activity. The optical heart activity sensor may detect the user's 100 heart activity by optical heart rate measurement, which may comprise sending a light beam towards user's 100 skin and measuring the bounced and/or emitted light from the user's 100 skin. The light beam may alter when travelling through the user's 100 veins and the alterations may be detected by the optical heart rate activity sensor. By using the detected data, the wrist device 102, may determine user's 100 heart activity, such as pulse for example.

In an embodiment, the wrist device 102 comprises a motion sensor configured to measure motion induced by the user 100 to the wrist device 102 by moving hand in which the user 100 wears the wrist device 102. The motion sensor may use other motion data, such as location data of the user, to determine user's 100 motion.

In an embodiment, the motion sensor(s) comprise at least one of the following: an accelerometer, a magnetometer, and a gyroscope.

In an embodiment, the motion sensor comprises an accelerometer and a gyroscope. The motion sensor may further comprise sensor fusion software for combining the accelerometer data and gyroscope data so as to provide physical quantities, such as acceleration data, velocity data, or limb trajectory data in a reference coordinate system having orientation defined by a predetermined gyroscope orientation.

In an embodiment, the motion sensor comprises a gyroscope and a magnetometer. The motion sensor may further comprise sensor fusion software to combine gyroscope data and magnetometer data so as to provide a reference coordinate system for the gyroscope based on the Earth magnetic field measured by the magnetometer. In general, the sensor fusion software described above may combine measurement data acquired from at least two motion sensors such that measurement data acquired from one motion sensor is used to establish the reference coordinate system for the measurement data acquired from at least one other motion sensor.

In an embodiment, the wrist device 102 comprises the motion sensor and the optical heart activity sensor.

In an embodiment, the wrist device 102 further comprises at least one of the following sensors: a temperature sensor, a positioning sensor and a pressure sensor. The positioning sensor may utilize GPS and/or Bluetooth information for locating the user 100. Naturally, the positioning sensor may comprise a magnetometer.

Still referring to Figure 1, the physical activity measurement system may further comprise external sensor devices 104 used by the user 100. The external sensor devices 104 may comprise sensors, such as a heart rate transmitter, a stride sensor, a positioning sensor, a cadence sensor and a power sensor, to mention a few. The heart rate transmitter may comprise at least one electrical and/or optical sensor to measure user's 100 heart activity. The positioning sensor may comprise a GPS, a magnetometer and/or a Bluetooth sensor. Thus, the positioning may be based on, for example, GPS location and/or Bluetooth location. The magnetometer may provide direction data based on magnetic fields on earth and/or inside structures.

The external sensor devices 104 may transmit the sensor data to the wrist device 102 and/or a portable electronic device 106 of the physical activity measurement system. The wrist device 102 and/or the portable electronic device 106 may receive the sensor data. The wrist device 102 and/or the portable electronic device 106 may comprise at least one processor configured to process the received sensor data, the heart activity data and/or motion data into a set of metrics describing the user's 100 physical activity. An example of the metrics may be seen in portable electronic device 106 listed as activity metric, pulse metric and route metric. The same set of metrics may be shown on the display of the wrist device 102.

The portable electronic device 106 may be a mobile phone, a smart phone, a palm device, a tablet computer, phablet or a portable digital assistant. The portable electronic device 106 and the wrist device 102 may be configured to establish a wireless communication connection with one another and exchange activity data, such heart activity data and motion data, over the wireless communication connection. The exchanged activity data may comprise the set of metrics described above. The wireless communication connection may utilize Near Field Communication (NFC), Radio Frequency Identification (RFID), Bluetooth, Bluetooth Low Energy, wireless local area network (WLAN), ANT or ANT+ by Dynastream, WIND 2.4 GHz or IEEE 802.15.4. Other short-range device-to-device or network communication protocols are equally possible. In an embodiment, the NFC circuitry is utilized to perform a handshake between the wrist device 102 and the portable electronic device 106, and other communication technologies, such as WiFi or Bluetooth, are used to transmit and/or receive the actual data to be transferred.

Sending the activity data to the portable electronic device 106 may be beneficial, as the display of the portable electronic device 106 may be bigger and may have a higher display resolution. The user interface of the portable electronic device 106 may also be more efficient to use compared to the interface of the wrist device 102.

However, performing physical activities may be more difficult when the portable electronic device 106 is carried by the user 100 during the physical activity. The wrist device 102 may be easier to carry with during all kinds of physical activities. Also the wrist device 102 may be worn all day long with ease when performing normal daily routines or activities, such as sitting, walking, driving, to name a few. This may enable the wrist device 102 to measure and display user's 100 physical activity and also inactiveness. Thus, to enable better and more efficient metric monitoring directly from the wrist device 102, the user interface of the wrist device 102 may need to be enhanced. Another aspect may be the visual brilliance of the device to which the user interface may have an effect. By enhancing the wrist device's 102 physical activity measurement and the possibility of the user 100 to monitor the physical activity-related metric the overall user experience may enhanced and the attractiveness of the physical activity measurement may be increased.

Figure 2A illustrates the wrist device 102 of Figure 1 according to an embodiment of the invention. Referring to Figure 2A, the wrist device 102 may comprise a curved body 202 arranged to fit the wrist device 102 around the user's 100 wrist. The wrist device 102 may comprise at least two connection members 208 arranged to connect the wrist device 102 around the user's 100 wrist by closing the loop formed by the wrist device 102. The at least two connection member 208 may be situated at the two ends of a wrist strap comprised in the wrist device 102. The curved body 202 may comprise the said wrist strap.

The wrist device 102 may comprise an activity tracker circuitry 210 configured to measure physical activity of the user 100 of the wrist device 102. The activity tracker circuitry 210 may comprise the optical heart activity sensor 212 and/or the motion sensor 214 described in with more detail in relation to Figure 1. The wrist device 102 may further comprise a processing circuitry 206 configured to acquire physical activity-related measurement data from the activity tracker circuitry 210 and to process the physical activity-related measurement data into physical activity-related metric characterizing the physical activity of the user. Furthermore, the wrist device 102 may comprise a wireless communication circuitry, wherein the wireless communication circuitry, enabling the wireless communication connection described above, may receive sensor data from the external sensor devices 104. The physical activity-related metric may be further based on the received external sensor data, provided by the external sensor devices 104. The physical activity-related metric may comprise the set of metrics, shown in the portable electronic device 106 of Figure 1.

In an embodiment, the activity tracker circuitry 210 comprises a bioimpedance sensor, wherein the bioimpedance sensor is configured to measure user's 100 heart activity. The measurement may be based on transmitting a radio signal into user's 100 skin, and observing changes in the radio signal due to impedance changes caused by, for example, blood volume changes. Thus, the user's 100 heart activity, such as heart rate, may be determined by the processing circuitry 206 from the data produced by the bioimpedance sensor. The bioimpedance sensor may be also used to measure and/or estimate, for example, the amount of water in user's 100 body.

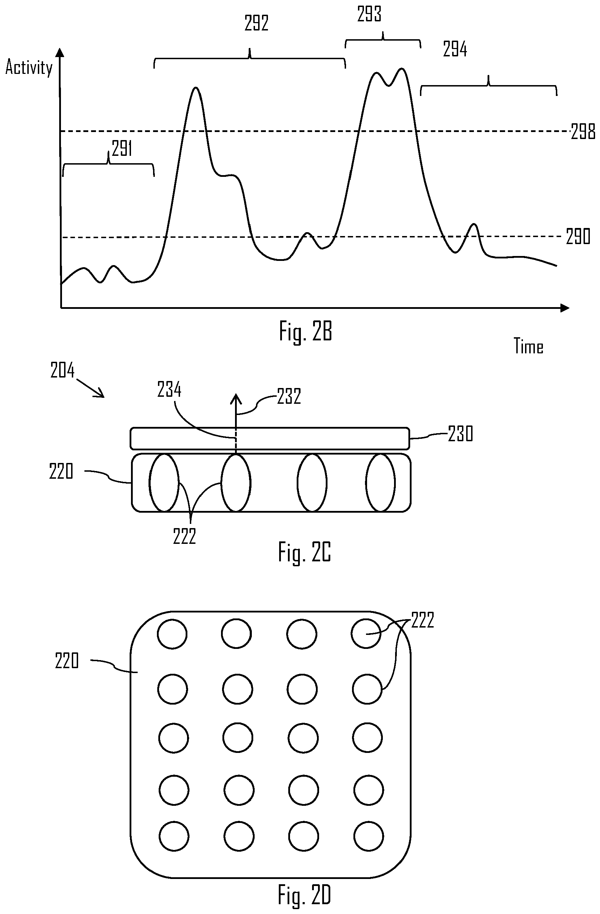

Figure 2B illustrates the measuring of activity according to an embodiment of the invention. The user's 100 activity may be illustrated in relation to time. Referring to Figure 2B, the user's 100 activity may be shown with a graph comprising parts 291-294. The different graph parts 291-294 may illustrate different times of a day. The said graph may illustrate a one full day, for example. The said graph may illustrate a user's 100 physical activity, wherein the physical activity may be measured by the activity tracker circuitry 210 and/or the external sensor devices 104. The measured physical activity may be further processed by the processing circuitry 206, based on the information provided by the activity tracker circuitry 210 and/or the external sensor devices 104. Based on the measured physical activity, the processing circuitry 206 may process the measured information into physical activity related metric.

The processing circuitry 206 may determine, based on the measure physical activity that the first part 291 of the activity graph may be under the inactivity threshold 290. The processing circuitry 206 may further determine that the user 100 is in sleep during the first part 291. It may also be possible, for the processing circuitry 206, to determine different stages of sleep, such rapid eye movement sleep (REM). The determination of sleep and/or different stages of sleep may be based on the measured physical activity information, such as heart activity and movement of the user 100 during sleep, for example. It may be also possible to determine, by the processing circuitry 210, the quality of sleep using the data from the different sensors mentioned earlier. The processing circuitry 210 may cause the display of the wrist device 102 to display the processed information, such as the sleep information.

The processing circuitry 210 may determine that second part 292 comprises parts where the measured physical activity of the user 100 may be over the activity threshold 298. When the physical activity may be over the activity threshold 298, the user 100 may be determined, by the processing circuitry 206, to be performing a physical activity. The second part 292 may be the time the user 100 is at work and/or going to work. Thus, the activity peak may be a bicycle ride to work, for example. Furthermore, the inactivity periods of the second part 292 may be determined to be time that is spent sitting by the user 100.

The third part 293 may comprise a part spent by the user 100 performing a physical exercise, such as running, for example. The measured physical activity may be substantially over the activity threshold 298 as the physical activity may be quite exhausting for the user 100. The processing circuitry 206 may determine that the third part 293 comprises the physical exercise and/or the user 100 may provide the information of the starting and/or stopping times of the physical exercise. The fourth part 294 may be determined by the processing circuitry 206 to comprise time spent sitting and sleeping. Thus, the fourth part 294 may be determined to represent evening activities of the user. The fourth part 294 may also comprise periods, wherein the activity is over the inactivity threshold 290.

The processing circuitry 206 may generate a physical activity metric based on the parts 291-294. The physical activity metric may comprise the cumulated physical activity of the user 100. Thus, the metric may show time spent over the activity threshold 298, the time spent below the inactivity threshold and/or the time spent between the thresholds 290, 298. The user interface element 204 may be configured to display the physical activity metric. The physical activity metric may be illustrated in a form of a circle diagram, for example. The activity and/or inactivity periods may be further illustrated with greater detail, wherein the periods may comprise information about sleep time, sitting time, physical exercise time and/or physical exercise type, for example. Therefore, the time spent for each activity may be shown.

Referring to Figure 2A, the wrist device 102 may comprise a user interface element 204 configured to display the physical activity-related metric, processed by the processing circuitry 206. The user interface element 204 may comprise a display to display the information and interface for the user 100 to interact with the information. Interaction may be possible with a mechanical keyboard, a virtual keyboard and/or other input means, such as touch screen selection or pointer. The pointer may be a virtual pen or user's 100 fingers, for example.

Looking closer to the user interface element 204, Figure 2C illustrates an embodiment of the user interface element 204. Referring to Figure 2C, the user interface element 204 may comprise a light emitting diode (LED) matrix layer 220 comprising a plurality of LEDs 222 arranged in a matrix form. The LED matrix layer 220 may comprise a film on which electric conductors are formed with a printing technique. The electric conductors may comprise installation areas for the plurality of LEDs 222. The said film may be a thin and flexible plastic film, for example.

In an embodiment, the LED matrix layer 220 comprises a printed circuit board (PCB), wherein the PCB is implemented with a thin and flexible plastic film on which electric conductors are formed with a printing technique. The flexible plastic film may have installation areas with conductors for the bare die LEDs 222, which may be inserted to the installation areas.

The user interface element 204 may further comprise a fluorescent layer 230 configured to alter the wavelength of the light emitted by the LED matrix layer 220. The fluorescent layer 230 may be situated on top of the LED matrix layer 220 so that the light emitted by the LED matrix layer 220 may be substantially emitted towards the fluorescent layer 230. The technical effect of the fluorescent layer 230 may be that the light, emitted by the LED matrix layer 220, may be seen as light with an altered wavelength at the surface of the fluorescent layer 230 giving an illusion that the displayed information may be right on the surface of the wrist device 102.

The use of fluorescent layer 230 may enable the use of LEDs 222 with different colors than the actual displayed color to the user 100. For example, if the LED matrix layer 220 emits blue light and the fluorescent layer 230 comprises yellow phosphor, the light perceived by the user may be substantially white light. This may mean that the perceived light may comprise photons perceived both as blue and yellow color. Thus, the combination of the two may be substantially white light seen on the surface of the fluorescent layer. The white light may enable the use of the whole spectrum of visible light as white light may be disassembled into different colors of the visible spectrum.

In an embodiment, the plurality of LEDs 222 are ultraviolet LEDs. Thus the LEDs 222 may emit substantially ultraviolet light.

In an embodiment, the plurality of LEDs 222 are blue LEDs. Thus the LEDs 222 may emit substantially blue light.

In an embodiment, the plurality of LEDs 222 comprise blue and ultraviolet LEDs. This may enable wider range of colors.

In an embodiment, the plurality of LEDs 222 are top-bottom bare LED dies. In such a scenario, the LEDs 222 may also be blue and/or ultraviolet LEDs.

In an embodiment, the plurality of LEDs 222 are red LEDs. In such a case the fluorescent layer may comprise material that may alter the red light wavelength into white light.

In an embodiment, the plurality of LEDs 222 are bare die LEDs.

The fluorescent layer 230 may increase the wavelength of the light emitted by the LED matrix layer 220 as the light travels through the fluorescent layer 230, which may be shown with an arrow 234 in Figure 2C. This may mean that the intensity of the light decreases. Thus, the color of the light may be different. The light that has travelled through the fluorescent layer 230 may be shown with an arrow 232 in Figure 2C.

In an embodiment, the fluorescent layer 230 comprises yellow phosphor. The yellow phosphor may be disposed unevenly within the fluorescent layer 230. This may mean that the phosphor may be disposed only to areas which are located substantially directly above the LEDs 222. Thus, the phosphor may be disposed to areas through which the light, emitted by the LED matrix layer 220, travels through.

In an embodiment, the fluorescent layer 230 is made of yellow phosphor.

In an embodiment, the fluorescent layer 230 is configured to decrease the wavelength of the light emitted by the LED matrix layer 220 travelling through the fluorescent layer 230.

Figure 2D shows the arrangement of plurality of LEDs 222 according to an embodiment of the invention. The Figure 2D may be understood as illustrating the LED matrix layer 220 from above compared to the side view of Figure 2C. Referring to Figure 2D, the plurality of LEDs 222 may be disposed in the LED matrix layer 220 forming the LED matrix, wherein the LED matrix may have certain amount of LEDs 222 in a row and a certain amount of LEDs 222 in a column. In the example of Figure 2D the LED matrix comprises five LED rows and four LED columns. Other amount of LED rows and columns may be equally possible. Each LED 222 in the LED matrix may be designed to illuminate in one or more colours. Thus, the LED matrix may be configured to display information for the user 100. Using fewer LEDs 222 may mean that the display resolution may be lower, but energy may be saved.

It needs to be noted that although Figures 2C and 2D illustrate a number of LEDs 222, only a few of the LEDs 222 are marked with a reference number.

In an embodiment, the size of the LED matrix formed by the plurality of LEDs 222 may be 5 x 34 which may equal to total 170 LEDs 222.

In an embodiment, the size of the LED matrix may be 5 x 17 which may equal to 85 LEDs 222.

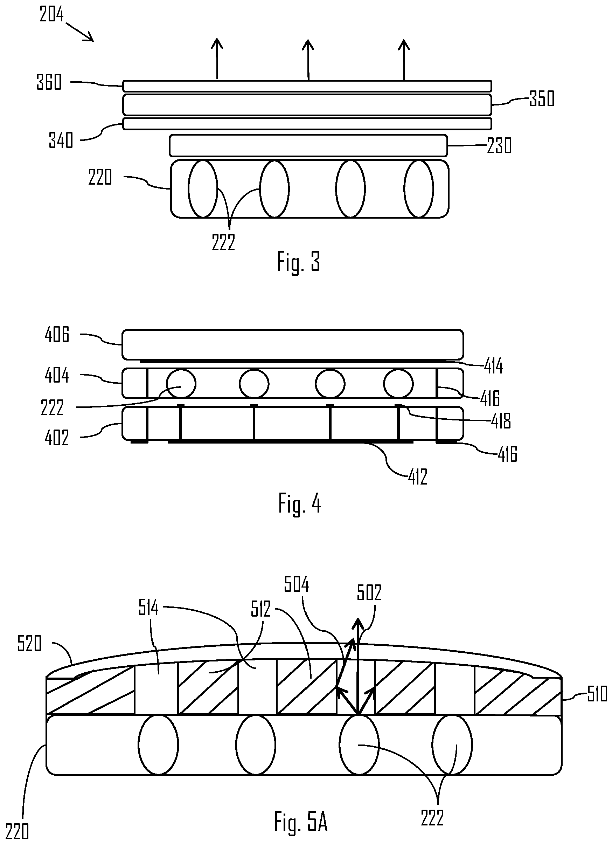

Figure 3 illustrates the user interface element 204 according to an embodiment of the invention. Referring to Figure 3, the user interface element 204 may comprise the LED matrix layer 220 and the fluorescent layer 230, as described earlier. The LED matrix layer 220 may further comprise a cover layer. The cover layer may act as a cover for the user interface element 204 and/or the wrist device 102. This may mean that the cover layer is casted on the wrist device 102, and thus may reach over the area of the user interface element 204. The cover layer may be a part of the curved body 202.

In an embodiment, the curved body 202 comprises and/or is made made from thermoplastic material, such as thermoplastic polyurethane (TPU).

In an embodiment, the cover layer is the surface of the interface element 204.

In an embodiment, the cover layer is arranged to be the top layer of the user interface element 204.

The cover layer may comprise a decoration layer 340, wherein the decoration layer may comprise a visual decoration, such as a product logo, a company logo, a scale indicator, dimmed areas, colored areas and/or instructions for the use of the user interface element 204. The instructions may comprise text indicating the function and/or meaning of a certain user interface element's 204 sub-element. The sub-element may be a mechanical and/or a virtual key, for example.

In an embodiment, the visual decoration of the decoration layer 340 is a dimming. The dimming may be arranged so that the light, emitted by the LED matrix layer 220, mostly travels through the dimming. The dimming may further intensify the visual brilliance of the user interface element 204 and/or the display formed by the layers 220, 230, 340, 350, 360 of the user interface element 204. The illusion of the contents right on the surface of the display may be further enhanced. The dimming may be a net-like structure, wherein the net-like structure comprises holes which allow the light to travel through. The net-like structure may be arranged so that it may block some light waves which may need to travel more distance. This may create a sharper outcome as light may come blurred the more distance it travels through different layers.

In an embodiment, the net-like structure is arranged so that the holes are situated above the LEDs 222.

In an embodiment, the dimming is achieved with a darkened foil.

In an embodiment, the dimming is achieved with a combination of the net-like structure and the darkened foil.

In an embodiment, the decoration layer 340 is made of a transparent and/or translucent material. The decorations may be done so that they are not directly above the LED matrix layer 220. This may mean that the decorations are done one the sides of the decoration layer. Thus, the decoration layer 340 may not be directly over the area where the display displays information.

In an embodiment, the decoration layer 340 is made of and/or comprises hardened glass. The hardened glass may be and/or comprise tempered glass, sapphire, clear ceramics, spinelor, aluminum oxynitride or laminated glass, for example.

In an embodiment, the decoration layer 340 is made of and/or comprises hardened plastic.

In an embodiment, the decoration layer 340 is arranged to be the bottom layer of the cover layer.

Still referring to Figure 3, the cover layer may comprise a lens layer 350. The lens layer 350 may act as a lens for the light emitted by the LED matrix layer 220 and altered by the fluorescent layer 230. The lens layer 350 may be made of material transparent and/or translucent to the light, and thus may not disturb the light traveling through. Furthermore, the lens layer 350 may be arranged to converge the light beams so that the information displayed to the user 100 may be sharper. This may mean that the light from the LEDs 222 may be more focused. The characteristics of the lens layer 350 may depend on the material used, curvature and thickens of the lens layer 350.

In an embodiment, the lens layer 350 is arranged to diverge light.

In an embodiment, the lens layer 350 is made of and/or comprises hardened glass. The hardened glass may be similar which was described in accordance with hardened glass used with the decoration layer 340.

In an embodiment, the lens layer 350 is made of and/or comprises hardened plastic. The hardened plastic may be and/or comprise, for example, polyethylene terephthalate (PET).

The cover layer may comprise a hardened layer 360. The hardened layer 360 may be made of transparent and/or translucent material. The material used may be hardened so that it may provide the user interface element 204 protection from external mechanical force, and thus protect the user interface element 204 and/or the wrist device 102.

In an embodiment, the hardened layer 360 is arranged to be the topmost layer of the cover layer.

In an embodiment, the hardened layer 360 is made of and/or comprises hardened glass. The hardened glass may be similar which was described in accordance with hardened glass used with the decoration layer 340.

In an embodiment, the hardened layer 360 is made of and/or comprises hardened plastic.

In an embodiment, the lens layer 350 is arranged to be situated between the decoration layer 340 and the hardened layer 360.

Figure 4 illustrates an embodiment of the invention. Referring to Figure 4, the LED matrix layer 220 may comprise the plurality of LEDs 222 as described earlier. The LED matrix layer 220 may further comprise a first layer 412 electrically coupled to an electrical terminal, such as a PCB. The first layer 412 may comprise electrical wiring which may enable the first layer to be connected to the LEDs 222. The wiring may go through a first film 402, and provide electrical connection points 418 to the LEDs 222.

In an embodiment, the first layer 412 and/or the first film may at least partially be manufactured with an electronics printing technique, such as sheet-based printing and/or roll-to-roll-based printing.

In an embodiment, the first film 402 comprises the first layer 412.

The LED matrix layer 220 may comprise a second layer 404. The second layer 404 may be a second film 404, for example. The second film 404 may comprise the plurality of LEDs 222 and/or the LEDs 222 may be disposed inside the second film 404. The plurality of LEDs 222 may be electrically coupled with the first layer 412. Electrical coupling may be enabled by the electrical connection points 418 to which the LEDs 222 may be connected.

In an embodiment, the second layer 404 may at least partially be manufactured with the electronics printing technique.

The LED matrix layer 220 may further comprise a third layer 414 electrically coupled with the plurality of LEDs 222. The first and second films 402, 404 may comprise lead-ins 416 which may be electrically coupled with the electrical terminal. Thus, an electrical circuit may be formed, wherein the LEDs 222 are provided with an operating voltage as the connection points 418 may complete the electrical circuit.

In an embodiment, the third layer 414 may at least partially be manufactured with the electronics printing technique.

In an embodiment, the electrical connection points 418 provide the LEDs 222 ground potential and the third layer 414 provides the LEDs 222 a potential different from the ground potential.

In an embodiment, the third layer 414 provides the LEDs 222 ground potential and the electrical connection points 418 provide potential different from the ground potential. The potentials different from ground potential may be negative and/or positive potentials, and thus the operating voltage may be negative and/or positive respectfully.

Still referring to Figure 4, the user interface element 204 may comprise a touch-screen layer 406 comprising a touch-screen element. The touch-screen layer 406 may be arranged to be situated between the LED matrix layer 220 and the cover layer described earlier. The touch-screen may enable the user to interact with the physical activity-related metric. The interaction may mean editing the metric, viewing the metric and/or causing the wrist device 102 to perform functions when the user 100 so chooses. The touch-screen layer 406 may comprise and/or be a third film.

In an embodiment, the user-interface element 204 comprises mechanical buttons that enable the user 100 to interact with the wrist device 102.

In an embodiment, the touch-screen element is a capacitive touch screen.

In an embodiment, the third film comprises the third layer 416. Thus, the touch-screen layer 406 may be comprised in the LED matrix layer 220.

In an embodiment, the LED matrix layer 220 comprises the touch-screen layer 406. The touch-screen layer 406 may be comprised in the third layer 414.

The layers 412, 404, 414 may be clued together. In parts where electrical coupling is needed, the glue used may need to be electricity conducting glue. In other parts the glue may not need to conduct electricity.

In an embodiment, the films 402, 404 and the touch-screen layer 406 are glued together.

In an embodiment, the first film 402, the second film 404 and/or the third film are polyethylene terephthalate PET films.

In an embodiment, the first film 402, the second film 404 and/or the third film comprise and/or are made from thermoplastic material, such as TPU.

In an embodiment, the first film 402, the second film 404 and/or the third film are each 0.2 millimeter (mm) thick.

In an embodiment, the first film 402, the second film 404 and/or the third film are transparent and/or translucent to the light emitted by the LEDs 222. This may mean that the films are transparent and/or translucent to, for example, blue and/or ultraviolet light.

In an embodiment, the LED matrix layer 220 is laminated inside a laminating structure. The laminating structure may comprise the first, second and/or third films described earlier.

In an embodiment, the first, second and third films form the earlier-mentioned PCB that is implemented with the thin and flexible plastic film. Thus, the PCB may comprise the first, second and third plastic films.

In an embodiment, the PCB comprises the first and second plastic films 402, 404.

Figure 5A illustrates the user interface element 204 according to an embodiment of the invention. Referring to Figure 5A, the user interface element 204 may comprise the LED matrix layer 220, wherein the LEDs 222 are disposed inside the LED matrix layer 220. The user interface element 204 may further comprise surface 520, wherein the surface 520 may comprise at least one of the following: fluorescent layer 230, the cover layer, the optical conductor layer 510 and the touch-screen layer 406.

In an embodiment the LEDs 222 are disposed substantially evenly inside the LED matrix layer.

The user interface element 204 may further comprise an optical conductor layer 510. The optical conductor layer 510 may enhance the contrast and the sharpness of the content displayed on the user interface element 204. This may be achieved by blocking and/or absorbing the light beams which have to travel more distance before are observed by the user 100. For example, if the layer on top of the LED matrix layer 220 would be the cover layer, the light beams which have a greater angle would have to travel in the cover layer more distance. This may mean that the light beams which travel on a greater angle may become blurred, and thus the content may become also blurred. The greater the distance for the light beams to travel, the more likely they may become blurred. Thus, the light from the LEDs 222 may be observed as blurred points, wherein light energy may be wasted as the intensity of the light beams may decrease and the light dots may increase in size. This may cause more energy to be used in order to display the activity-related metric with enough contrast and sharpness for the user 100. Thus, the optical conductor layer 510 may be needed to save energy and enabling the monitoring of physical activity for a longer time period.

The optical conductor layer 510 may comprise optical guides 514 and light guards 512. The optical guides 514 may comprise material which is transparent and/or translucent to the light beams emitted by the LEDs 222. An example of such material may be clear glass and/or clear plastic. In an embodiment, the material is colored glass and/or plastic. The light guards 512 may be made of colored glass and/or colored plastic which may block and/or absorb at least some of the light beams.

In an embodiment, the optical guides 514 are made of a substantially transparent material.

In an embodiment, the optical conductor layer 510 is arranged to be situated between the LED matrix layer 220 and the fluorescent layer 230.

In an embodiment, the optical conductor layer 510 is arranged to be situated between the fluorescent layer 230 and the cover layer. Thus, the optical conductor layer 510 may be situated, for example, between the fluorescent layer 230 and the decoration layer 440. The optical conductor layer 510 may also be situated under the touch-screen layer 406.

In an embodiment, the optical conductor layer 510 is configured to enhance the contrast of the user interface element by blocking at least some of the scattered light from the LED matrix layer 220. This may be achieved, for example, by the light guards 512 blocking at least some of the scattered light. The light guards 512 may further reflect some of the light back to the optical guides 514, and eventually to the user 100. The better contrast may help the user 100 to observe the activity-related metric in bright light conditions, for example in a bright sunlight.

Still referring to Figure 5A, the light emitted by the LEDs 222 may comprise light beams 502, wherein some of the light beams 502 may reach the surface 520 without touching any of the light guards 512, and eventually be observed by the user 100. Some other light beams 504 may be blocked, absorbed and/or reflected back to the optical guide 514. When the light beam 504 is reflected back to the optical guide 514, the light beam 504 may be observed by the user 100 the same way as the light beam 502.

In an embodiment, the optical conductor layer 510 comprises a plurality of optical guides 514 arranged in a matrix form, wherein the optical guides 514 are made of a substantially clear material, and wherein the plurality of optical guides 514 are arranged to be situated on top of the plurality of LEDs 222 so that the optical guides 514 are aligned with the LEDs 222. Thus, the light emitted by the LEDs 222 may be emitted towards the optical guides 514. Naturally, the light guards 512 may be also in matrix form, so that some of the light beams may be blocked, absorbed and/or reflected back to the optical guides 514, as described earlier.

In an embodiment, the optical conductor layer comprises a plurality of light guards 512 in a matrix form, wherein the light guards 512 are made of a substantially light blocking material, and wherein the plurality of light guards are arranged to be situated between the optical guides 514.

In an embodiment, the light guards 512 are further configured to reflect at least some of the light back to the optical guides 514.

In an embodiment, the user interface element 204 is and/or comprises a display. The display may be formed by at least one of the following: the LED matrix layer 220, the fluorescent layer 230, the cover layer and the optical conductor layer 510. The display may further comprise the touch-screen layer 406.

It needs to be noted that the embodiments shown in Figures 2B, 3, 4 and 5 may actually be curved as shown with user interface element 204 of Figure 2A. The not curved form illustrated in Figures 2B, 3, 4 and 5 has been done in sake of simplicity.

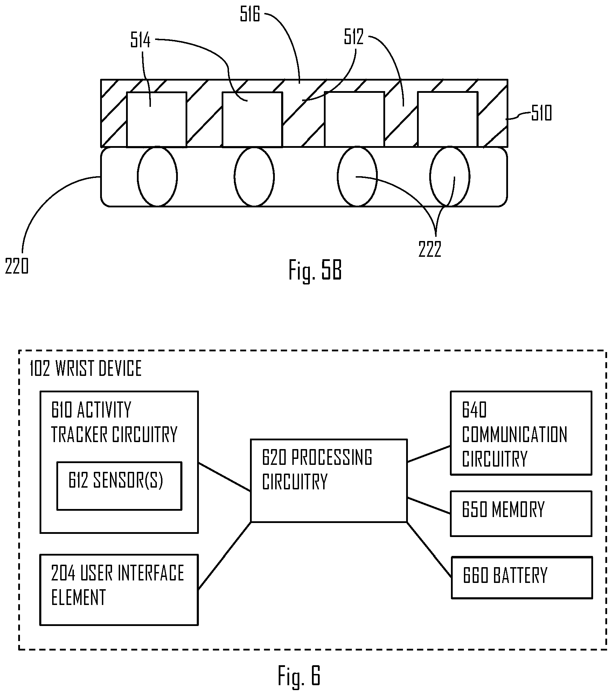

Figure 5B shows the optical conductor layer 510 according to an embodiment of the invention. The optical conductor layer 510 is shown in top of the LED matrix layer 220 to better illustrate the usage of the optical conductor layer 510. Referring to Figure 5B, the optical conductor layer 510 comprises a plurality of optical guides 514 and a plurality of light guards 512 as described in Figure 5A. The light guards 512 may be comprised in a light guard member 516. The light guard member 516 may form a comb-like structure and may be made of colored glass and/or colored plastic which may block and/or absorb at least some of the light beams emitted by the LEDs 222. The structure of the light guard member 516 may allow light beams to penetrate the light guard member 516 from the top part of the optical guides 514. This may happen because the light guard member 516 may be thinner from the areas which are adjacent to the optical guides 514 as shown in Figure 5B. Thus, even though the light guard member 516 may be arranged to block light, it may let the light beams to penetrate it from the areas where the optical guides 514 are located.

In an embodiment, the light guard member 516 is arranged to be upside down compared to the arrangement illustrated in Figure 5B. Thus, the thinner areas of the light guard member 516 may be located between the optical guides 514 and the LEDs 222.

In an embodiment, the optical conductor layer comprises a foil white layer arranged to be situated between the optical guides 514 and the LED matrix layer 220.

Figure 6 illustrates a block diagram of the wrist device 102 according to an embodiment of the invention. The wrist device 102 may comprise the activity tracker circuitry 610, such as activity tracker circuitry 210 described in Figure 2A. The activity tracker circuitry 610 may be configured to measure user's 100 physical activity. The activity tracker circuitry 610 may comprise sensor(s) 612, wherein the sensor(s) 612 may comprise the motion sensor 214 and/or the optical heart activity sensor 212. The sensor(s) 612 may further comprise sensors, such as a temperature sensor and a pressure sensor. The sensor(s) 612 may also comprise the external sensor devices 104, wherein the wrist device 102 may be connected to the external sensor devices via, for example, communication circuitry 640, wherein the communication circuitry 640 may comprise the elements of the communication circuitry described in regards to Figure 2A.

The wrist device 102 may further comprise the processing circuitry 620, such as the processing circuitry 206 described in Figure 2A, wherein the processing circuitry 620 is configured to acquire physical activity-related measurement data from the activity tracker circuitry and/or from external sensor devices 102, and to process the physical activity-related measurement data into physical activity-related metric characterizing the physical activity of the user 100. The activity-related metric may be, for example, burned calories, traveled distance and/or overall activity pie diagram. The activity pie diagram may show the time spent inactive, such as sitting, and the time spent active, such as walking or performing an exercise. The wrist device 102 may comprise the user interface element 204 configured to display the physical activity-related metric. Thus, different activity levels may be specified and displayed by the user interface element 204. Furthermore, the user 100 may interact with the physical activity-related metric via the user inter face element 204. This may be possible as the user interface element 204 may comprise a touch-screen panel and/or physical interface for the user to input and/or edit information.

The wrist device 102 may further comprise a memory 650, wherein the memory 650 may comprise software, such as operating system, of the wrist device 102. The memory 650 may be used to save training files and/or physical activity-related information, which may be transferred later to an external device and/or a service.

The wrist device 102 may also comprise a battery 660 arranged to provide the wrist device 102 an operating voltage. The battery 660 may provide the LEDs 222 an operating voltage. As described earlier, used energy and eventually the battery 660 may be saved by using the described solution in the wrist device 102.

As used in this application, the term 'circuitry' refers to all of the following: (a) hardware-only circuit implementations, such as implementations in only analog and/or digital circuitry, and (b) combinations of circuits and soft-ware (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network device, or another network device.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described herein, or a combination thereof. For firmware or software, the implementation can be carried out through modules of at least one chip set (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, described with regard thereto, and they are not limited to the precise configurations set forth in the given figures, as will be appreciated by one skilled in the art.

Embodiments as described may also be carried out in the form of a computer process defined by a computer program. The computer program may be in source code form, object code form, or in some intermediate form, and it may be stored in some sort of carrier, which may be any entity or device capable of carrying the program. For example, the computer program may be stored on a computer program distribution medium readable by a computer or a processor. The computer program medium may be, for example but not limited to, a record medium, computer memory, read-only memory, electrical carrier signal, telecommunications signal, and software distribution package, for example. Coding of software for carrying out the embodiments as shown and described is well within the scope of a person of ordinary skill in the art.

Even though the invention has been described above with reference to an example according to the accompanying drawings, it is clear that the invention is not restricted thereto but can be modified in several ways within the scope of the appended claims.

## Claims

1. A wrist device (102) comprising:
a curved body (202) arranged to fit the wrist device around a user's wrist;
an activity tracker circuitry (210) configured to measure physical activity of the user of the wrist device;
a processing circuitry (206) configured to acquire physical activity-related measurement data from the activity tracker circuitry (210) and to process the physical activity-related measurement data into physical activity-related metric characterizing the physical activity of the user; and
a user interface element (204) configured to display the physical activity-related metric, wherein the user interface element (204) comprises:
a light emitting diode matrix layer (220) comprising a plurality of light emitting diodes (222) arranged in a matrix form, **characterised in that** the wrist device further comprises
a fluorescent layer (230) configured to alter the wavelength of the light emitted by the light emitting diode matrix layer,
wherein the light emitting diode matrix layer (220) comprises: a first layer (412) electrically coupled to an electrical terminal, a second layer (404) comprising the plurality of light emitting diodes (222), wherein the plurality of light emitting diodes (222) are electrically coupled with the first layer (412), and a third layer (414) electrically coupled with the plurality of light emitting diodes (222), wherein the second layer (404) comprises electrical lead-troughs (416) electrically coupling the first layer (412) and the third layer (414),
and wherein at least one of the first layer (412), the second layer (404), and the third layer (414) is manufactured by utilizing electronics printing technique.

2. The wrist device of claim 1, wherein the user interface element further comprises an optical conductor layer (510), and wherein the optical conductor layer is configured to enhance the contrast of the user interface element by blocking at least some of the scattered light from the light emitting diode matrix layer.

3. The wrist device of claim 2, wherein the optical conductor layer is arranged to be situated between the light emitting diode matrix layer and the fluorescent layer.

4. The wrist device of any of the claims 2 to 3, wherein the optical conductor layer comprises a plurality of optical guides (514) arranged in a matrix form, wherein the optical guides are made of a substantially transparent material to the wavelength of the plurality of light emitting diodes, and wherein the plurality of optical guides are arranged to be situated on top of the plurality of light emitting diodes so that the optical guides are aligned with the light emitting diodes.

5. The wrist device of claim 4, wherein the optical conductor layer further comprises a plurality of light guards (512) in a matrix form, wherein the light guards are made of a substantially light blocking material, and wherein the plurality of light guards are arranged to be situated between the optical guides.

6. The wrist device of claim 5, wherein the light guards are further configured to reflect at least some of the light back to the optical guides.

7. The wrist device of any preceding claim, wherein the light emitting diode matrix layer is laminated inside a laminating structure.

8. The wrist device of any preceding claim, wherein the fluorescent layer is configured to increase the wavelength of the light emitted by the light emitting diode matrix layer.

9. The wrist device of any preceding claim, wherein the fluorescent layer is arranged to be situated on top of the light emitting diode matrix layer.

10. The wrist device of any preceding claim, wherein the user interface element further comprises a cover layer arranged to be the top layer of the user interface element.

11. The wrist device of claim 10, wherein the cover layer comprises a hardened layer arranged to be the topmost layer of the cover layer, and wherein the hardened layer is made of transparent material providing the user interface element protection from external mechanical force.

12. The wrist device of any of claims 10 to 11, wherein the cover layer comprises a decoration layer arranged to be the bottom layer of the cover layer, and wherein the decoration layer comprises a visual decoration.

13. The wrist device of any preceding claim, wherein the user interface element comprises a touch-screen layer comprising a touch-screen element, and wherein the touch-screen layer is arranged to be situated between the light emitting diode matrix layer and the cover layer.

14. The wrist device of claim 1, wherein the light emitting diode matrix layer comprises a thin and flexible plastic film on which electric conductors are formed with a printing technique, and wherein the electric conductors comprise installation areas for the plurality of light emitting diodes.

## Patentansprüche

1. Handgelenksvorrichtung (102), die Folgendes umfasst:
einen gekrümmten Körper (202), der angeordnet ist, die Handgelenksvorrichtung um das Handgelenk eines Benutzers anzupassen;
eine Aktivitätsverfolgerschaltung (210), die dazu ausgelegt ist, eine physische Aktivität des Benutzers der Handgelenksvorrichtung zu messen;
eine Verarbeitungsschaltung (206), die dazu ausgelegt ist, eine physische Aktivität betreffende Messdaten von der Aktivitätsverfolgerschaltung (210) zu erfassen und die eine physische Aktivität betreffenden Messdaten zu einer eine physische Aktivität betreffende Metrik zu verarbeiten, die die physische Aktivität des Benutzers charakterisiert; und
ein Benutzerschnittstellenelement (204), das dazu ausgelegt ist, die eine physische Aktivität betreffende Metrik anzuzeigen, wobei das Benutzerschnittstellenelement (204) Folgendes umfasst:
eine lichtemittierende Diodenmatrixschicht (220) die eine Vielzahl von lichtemittierenden Dioden (222) umfasst, die in einer Matrix angeordnet sind, **dadurch gekennzeichnet, dass** die Handgelenksvorrichtung ferner Folgendes umfasst
eine fluoreszierende Schicht (230), die dazu ausgelegt ist, die Wellenlänge des Lichts das von der lichtemittierenden Diodenmatrixschicht emittiert wird, zu ändern,
wobei die lichtemittierende Diodenmatrixschicht (220) Folgendes umfasst: eine erste Schicht (412), die elektrisch an ein elektrisches Endgerät gekoppelt ist, eine zweite Schicht (404), die eine Vielzahl von lichtemittierenden Dioden (222) umfasst, wobei die Vielzahl von lichtemittierenden Dioden (222) elektrisch an die erste Schicht (412) gekoppelt sind, und eine dritte Schicht (414), die an die Vielzahl von lichtemittierenden Dioden (222) gekoppelt ist, wobei die zweite Schicht (404) elektrische Durchführungen (416) umfasst, die die erste Schicht (412) elektrisch an die dritte Schicht (414) koppelt,
und wobei mindestens eine der ersten Schicht (412), der zweiten Schicht (404) und der dritten Schicht (414) unter Einsatz einer elektronischen Drucktechnik hergestellt wird.

2. Handgelenksvorrichtung nach Anspruch 1, wobei das Benutzerschnittstellenelement ferner eine optische Leiterschicht (510) umfasst und wobei die optische Leiterschicht dazu ausgelegt ist, den Kontrast des Benutzerschnittstellenelements durch Blockieren von mindestens einigem des Streulichts von der lichtemittierenden Diodenmatrixschicht zu verbessern.

3. Handgelenksvorrichtung nach Anspruch 2, wobei die optische Leiterschicht angeordnet ist, zwischen der lichtemittierenden Diodenmatrixschicht und der fluoreszierenden Schicht platziert zu sein.

4. Handgelenksvorrichtung nach einem der Ansprüche 2 bis 3, wobei die optische Leiterschicht eine Vielzahl von optischen Führungen (514) umfasst, die in einer Matrixform angeordnet sind, wobei die optischen Führungen aus einem für die Wellenlänge der Vielzahl von lichtemittierenden Dioden im Wesentlichen transparenten Material bestehen, und wobei die Vielzahl von optischen Führungen angeordnet sind, auf der Vielzahl von lichtemittierenden Dioden platziert zu sein, derart, dass die optischen Führungen auf die lichtemittierenden Dioden ausgerichtet sind.

5. Handgelenksvorrichtung nach Anspruch 4, wobei die optische Leiterschicht ferner eine Vielzahl von Lichtschranken (512) in einer Matrixform umfasst, wobei die Lichtschranken aus einem im Wesentlichen lichtblockierendem Material bestehen, und wobei die Vielzahl von Lichtschranken angeordnet sind, zwischen den optischen Führungen platziert zu sein.

6. Handgelenksvorrichtung nach Anspruch 5, wobei die Lichtschranken ferner dazu ausgelegt sind, mindestens einiges des Lichts zu den optischen Führungen zurück zu reflektieren.

7. Handgelenksvorrichtung nach einem der vorhergehenden Ansprüche, wobei die lichtemittierende Diodenmatrixschicht in einer laminierenden Struktur laminiert ist.

8. Handgelenksvorrichtung nach einem der vorhergehenden Ansprüche, wobei die fluoreszierende Schicht dazu ausgelegt ist, die Wellenlänge des Lichts, das von der lichtemittierenden Diodenmatrixschicht emittiert wird, zu erhöhen.

9. Handgelenksvorrichtung nach einem der vorhergehenden Ansprüche, wobei die fluoreszierende Schicht angeordnet ist, auf der lichtemittierenden Diodenmatrixschicht platziert zu sein.

10. Handgelenksvorrichtung nach einem der vorhergehenden Ansprüche, wobei das Benutzerschnittstellenelement ferner eine Deckschicht umfasst, die angeordnet ist, die obere Schicht des Benutzerschnittstellenelements zu sein.

11. Handgelenksvorrichtung nach Anspruch 10, wobei die Deckschicht eine gehärtete Schicht umfasst, die angeordnet ist, die oberste Schicht der Deckschicht zu sein, und wobei die gehärtete Schicht aus transparentem Material besteht, das das Benutzerschnittstellenelement mit einem Schutz vor einer äußeren mechanischen Kraft versieht.

12. Handgelenksvorrichtung nach einem der Ansprüche 10 bis 11, wobei die Deckschicht eine Dekorationsschicht umfasst, die angeordnet ist, die untere Schicht der Deckschicht zu sein, und wobei die Dekorationsschicht eine optische Dekoration umfasst.

13. Handgelenksvorrichtung nach einem der vorhergehenden Ansprüche, wobei das Benutzerschnittstellenelement eine Touchscreenschicht umfasst, die ein Touchscreenelement umfasst, und wobei die Touchscreenschicht angeordnet ist, zwischen der lichtemittierenden Diodenmatrixschicht und der Deckschicht platziert zu sein.

14. Handgelenksvorrichtung nach Anspruch 1, wobei die lichtemittierende Diodenmatrixschicht eine dünne und flexible Kunststofffolie umfasst, auf der elektrische Leiter mit einer Drucktechnik gebildet sind, und wobei die elektrischen Leiter Installationsbereiche für die Vielzahl von lichtemittierenden Dioden umfassen.

## Revendications

1. Dispositif de poignet (102) qui comprend :
un corps incurvé (202) agencé pour ajusté le dispositif de poignet autour du poignet d'un utilisateur ;
des éléments de circuit de suivi d'activité (210) configurés pour mesurer l'activité physique de l'utilisateur du dispositif de poignet ;
des éléments de circuit de traitement (206) configurés pour acquérir des données de mesure associées à l'activité physique à partir des éléments de circuit de suivi d'activité (210) et pour traiter les données de mesure associées à l'activité physiques en une métrique associée à l'activité physique qui caractérise l'activité physique de l'utilisateur ; et
un élément d'interface utilisateur (204) configuré pour afficher la métrique associée à l'activité physique, dans lequel l'élément d'interface utilisateur (204) comprend :
une couche de matrice de diodes électroluminescentes (220) qui comprend une pluralité de diodes électroluminescentes (222) agencées en une matrice, **caractérisé en ce que** le dispositif de poignet comprend en outre :
une couche fluorescente (230) configurée pour modifier la longueur d'onde de la lumière émise par la couche de matrice de diodes électroluminescentes,
dans lequel la couche de matrice de diodes électroluminescentes (220) comprend : une première couche (412) couplée électriquement à une borne électrique, une deuxième couche (404) qui comprend la pluralité de diodes électroluminescentes (222), dans lequel la pluralité de diodes électroluminescentes (222) sont couplées électriquement à la première couche (412), et une troisième couche (414) couplée électriquement à la pluralité de diodes électroluminescentes (222), dans lequel la deuxième couche (404) comprend des traversées électriques (416) qui couplent électriquement la première couche (412) et la troisième couche (414),
et dans lequel au moins l'une de la première couche (412), de la deuxième couche (404), et de la troisième couche (414) est fabriquée en utilisant une technique d'impression électronique.

2. Dispositif de poignet selon la revendication 1, dans lequel l'élément d'interface utilisateur comprend en outre une couche de conduction optique (510), et dans lequel la couche de conduction optique est configurée pour améliorer le contraste de l'élément d'interface utilisateur en bloquant au moins une partie de la lumière diffusée qui provient de la couche de matrice de diodes électroluminescentes.

3. Dispositif de poignet selon la revendication 2, dans lequel la couche de conduction optique est agencée de manière à être située entre la couche de matrice de diodes électroluminescentes et la couche fluorescente.

4. Dispositif de poignet selon l'une quelconque des revendications 2 à 3, dans lequel la couche de conduction optique comprend une pluralité de guides optiques (514) agencés en une forme matricielle, dans lequel les guides optiques sont constitués d'un matériau sensiblement transparent à la longueur d'onde de la pluralité de diodes électroluminescentes, et dans lequel la pluralité de guides optiques sont agencés de manière à être situés sur la pluralité de diodes électroluminescentes de sorte que les guides optiques soient alignés avec les diodes électroluminescentes.

5. Dispositif de poignet selon la revendication 4, dans lequel la couche de conduction optique comprend en outre une pluralité d'écrans à la lumière (512) en une forme matricielle, dans lequel les écrans à la lumière sont constitués d'un matériau bloquant sensiblement la lumière, et dans lequel la pluralité d'écrans à la lumière sont agencés de manière à être situés entre les guides optiques.

6. Dispositif de poignet selon la revendication 5, dans lequel les écrans à la lumière sont en outre configurés pour réfléchir au moins une partie de la lumière de retour vers les guides optiques.

7. Dispositif de poignet selon l'une quelconque des revendications précédentes, dans lequel la couche de matrice de diodes électroluminescentes est lamifiée à l'intérieur d'une structure de lamification.

8. Dispositif de poignet selon l'une quelconque des revendications précédentes, dans lequel la couche fluorescente est configurée pour augmenter la longueur d'onde de la lumière émise par la couche de matrice de diodes électroluminescentes.

9. Dispositif de poignet selon l'une quelconque des revendications précédentes, dans lequel la couche fluorescente est agencée de manière à être située sur la couche de matrice de diodes électroluminescentes.

10. Dispositif de poignet selon l'une quelconque des revendications précédentes, dans lequel l'élément d'interface utilisateur comprend en outre une couche de recouvrement agencée de manière à être la couche supérieure de l'élément d'interface utilisateur.

11. Dispositif de poignet selon la revendication 10, dans lequel la couche de recouvrement comprend une couche durcie agencée de manière à être la couche la plus haute de la couche de recouvrement, et dans lequel la couche durcie est constituée d'un matériau transparent qui assure la protection de l'élément d'interface utilisateur contre une force mécanique externe.

12. Dispositif de poignet selon l'une quelconque des revendications 10 à 11, dans lequel la couche de recouvrement comprend une couche de décoration agencée de manière à être la couche inférieure de la couche de recouvrement, et dans lequel la couche de décoration comprend une décoration visuelle.

13. Dispositif de poignet selon l'une quelconque des revendications précédentes, dans lequel l'élément d'interface utilisateur comprend une couche d'écran tactile qui comprend un élément d'écran tactile, et dans lequel la couche d'écran tactile est agencée de manière à être située entre la couche de matrice de diodes électroluminescentes et la couche de recouvrement.

14. Dispositif de poignet selon la revendication 1, dans lequel la couche de matrice de diodes électroluminescentes comprend un film en matière plastique mince et souple sur lequel des conducteurs électriques sont formés par une technique d'impression, et dans lequel les conducteurs électriques comprennent des zones d'installation pour la pluralité de diodes électroluminescentes.
